# EUROPEAN PATENT APPLICATION

(11) **EP 3 957 634 A1**
(43) Date of publication of application: **23.02.2022**
(21) Application number: 20836320.0
(22) Date of filing: 03.07.2020
(51) Int. Cl.: C07D 407/10, C07H 1/00, A61P 3/10

(54) **CRYSTAL FORM OF SGLT INHIBITOR AND APPLICATION THEREOF**

(30) Priority: 05.07.2019 CN 201910604504
(71) Applicant: Shangdong Danhong Pharmaceutical Co., Ltd., Shangdong 274000 (CN)
(72) Inventor: MAO, Qinghua, Shanghai 200131 (CN); YU, Tao, Shanghai 200131 (CN); LI, Yi, Shanghai 200131 (CN); WU, Chengde, Shanghai 200131 (CN); YAO, Ting, Shanghai 200131 (CN)
(74) Representative: Groth & Co. KB
(86) International application number: PCT/CN2020/100072
(87) International publication number: WO 2021/004388

(57) **Abstract**

Crystal form A of a compound represented by formula (I) and an application thereof in preparing a drug for treating an SGLT1/SGLT2-related disease.

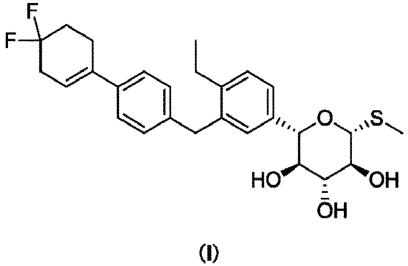

## Description

The present application claims the following priority:
CN 201910604504.7, filed on July 5, 2019.

### TECHNICAL FIELD

The present invention relates to crystal form A of a compound represented by formula (I) and use thereof in the manufacture of a medicament for treating SGLT1/SGLT2 related diseases.

### BACKGROUND OF THE INVENTION

Diabetes is a metabolic disease characterized by hyperglycemia. Hyperglycemia is caused by insulin secretion defects or damage to their biological action, or both. In case of diabetes, the long-term blood glucose level abnormality can lead to severe complications, including cardiovascular diseases, chronic renal failure, retinal damage, nerve injury, microvascular injury, obesity and the like. For treatment of diabetes, diet control and exercise therapy are preferred glycemic control schemes at early stage. When these methods are difficult to achieve the control of blood glucose, it is necessary to use insulin or oral hypoglycemic drugs for treatment. Currently, a variety of hypoglycemic drugs are used for clinical treatment, mainly including biguanides, sulfonylurea, insulin tolerance improving agents, glinides, alpha-glucosidase inhibitors, dipeptidyl peptidase-IV inhibitors, and the like. These drugs have a good therapeutic effect, but there remains a safety problem for long-term treatment, for example, biguanides are susceptible to lactic acid poisoning; sulfonylurea can lead to hypoglycemia symptoms; insulin tolerance improving agents can cause edema, heart failure and weight gain; alpha-glucosidase inhibitors can cause symptoms such as abdominal pain, abdominal distension, diarrhea and the like. Therefore, there is an urgent need to develop a novel hypoglycemic drug that is safer and more effective to meet the therapeutic requirements of diabetes.

Sodium-glucose cotransporters (SGLTs) belong to a family of glucose transporter found in small intestinal mucosa and kidney proximal tubule, and the family members mainly include two classes of SGLT1 protein and SGLT2 protein, function of which is to mediate transmembrane transport of glucose in intestinal tract and kidney, playing a key role in maintaining the stability of human blood glucose. Specifically, SGLT1 is mainly distributed in intestinal mucosa cells of the small intestine, with a small amount of expression in the myocardium and the kidney, and mainly regulates the intestinal absorption process of glucose. In addition, SGLT2 is highly expressed in the kidney, and is mainly responsible for the regulation of the glucose renal reuptake process, that is, glucose in urine can be actively attached to the renal tubular epithelial cells and transported into the cells by SGLT2 protein for reuse during glomerular filtration. In this process, SGLT2 is responsible for 90% of the reabsorption process, and the remaining 10% is completed by SGLT1. Since this process does not intervene in the metabolism of glucose, the occurrence of adverse reactions of hypoglycemia is avoided or reduced, and the risk of causing cardiovascular diseases is reduced. Therefore, SGLTs has become one of the ideal potential targets for treating diabetes.

In view of this, some SGLTs inhibitors, especially highly selective SGLT2 inhibitors, are successively developed. They specially inhibit the reabsorption of glucose in the kidney by inhibiting SGLT2 activity, thereby increasing the excretion of glucose in urine and normalizing plasma glucose in diabetic patients. Since 2012, six drugs including Dapagliflozin, Canagliflozin, Luseogliflozin, Itragliflozin, Tofogliflozin, and Enagliflozin have been approved for the market and become effective drugs for treating diabetes.

In addition to the selective SGLT2 inhibitor, it has been found in recent years that partial inhibition of SGLT1 while SGLT2 is inhibited can not only inhibit the reuptake of glucose in the kidney but also control the absorption of glucose in the intestinal tract without occurrence of diarrhea or other gastrointestinal reactions. Meanwhile, glucose from the gastrointestinal tract to blood is reduced by inhibiting intestinal SGLT1, so that the postprandial GLP -1 and the PYY level can be increased, thereby showing better hypoglycemic effects than the selective SGLT2 inhibitor and reducing the risk of urinary tract infection and renal function damage and the like. Thus, the development of SGLT-1/SGLT2 dual inhibitors has become a new target and direction for diabetes treatment in recent years.

In summary, as a new type of drug for diabetes treatment, the SGLT1/SGLT2 dual inhibitor has a good development prospect. Therefore, it is urgently needed to develop an SGLT1/SGLT2 dual inhibitor with good efficacy, good pharmacokinetic properties and high safety for the treatment of diabetes and related metabolic disorder diseases. At present, Sotagliflozin, which is a SGLT1/SGLT2 dual inhibitor developed by Lexicon Corporation and Sanofi Corporation, has completed clinical III-phase studies (WO 2008042688/WO2012094293).

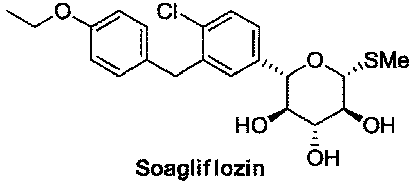

### SUMMARY OF THE INVENTION

Provided in the present invention is a crystal form A of a compound represented by formula (I), wherein an X-ray powder diffraction pattern of the crystal form A has characteristic diffraction peaks at the following 2θ angles: 5.09 ± 0.20°, 9.35 ± 0.20°, and 13.24 ± 0.20°

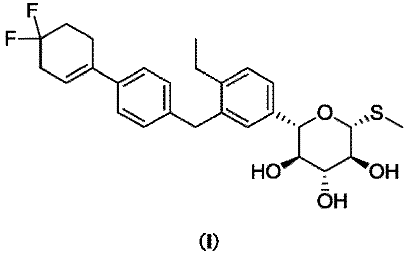

In some embodiments of the present invention, the X-ray powder diffraction pattern of the crystal form A has characteristic diffraction peaks in the following 2θ angles: 5.09 ± 0.20°, 9.35 ± 0.20°, 13.24 ± 0.20°, 14.35 ± 0.20°, 15.86 ± 0.20°, 17.04 ± 0.20°, 18.04 ± 0.20°, and 19.16 ± 0.20°.

In some embodiments of the present invention, the X-ray powder diffraction pattern of the above crystal form A has characteristic diffraction peaks at the following 2θ angles: 5.09 ± 0.20°, 7.13 ± 0.20°, 9.35 ± 0.20°, 11.98 ± 0.20°, 13.24 ± 0.20°, 14.35 ± 0.20°, 15.86 ± 0.20°, 17.04 ± 0.20°, 18.04 ± 0.20°, and 19.16 ± 0.20°.

In some embodiments of the present invention, the X-ray powder diffraction pattern of the above crystal form A has characteristic diffraction peaks at the following 2θ angles: 3.33°, 4.84°, 5.09°, 7.13°, 9.35°, 10.17°, 11.98°, 13.24°, 14.35°, 14.62°, 15.38°, 15.86°, 17.04°, 18.04°, 19.16°, 19.95°, 20.26°, 20.70°, 21.71°, 22.52°, 24.43°, 26.11° and 28.41°.

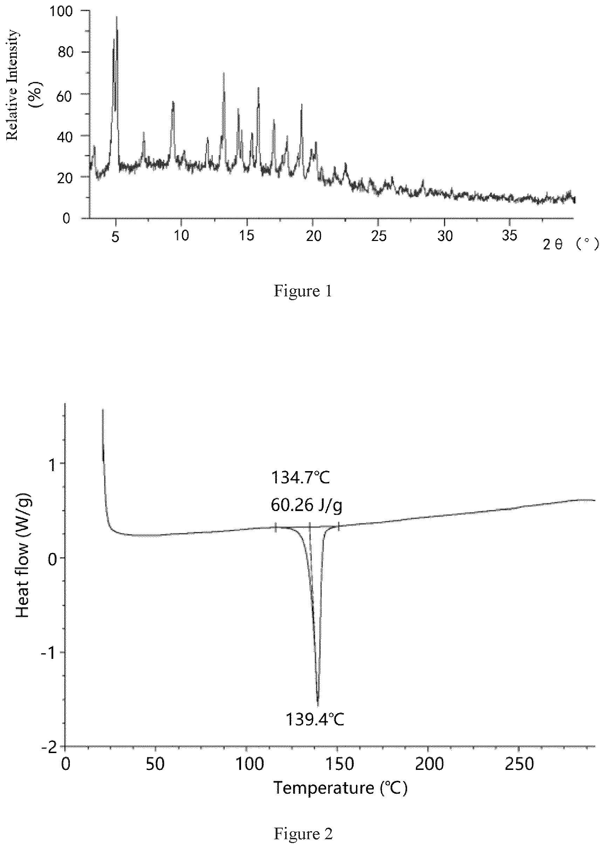

In some embodiments of the present disclosure, the XRPD pattern of the crystal form A is as shown in Figure 1.

In some embodiments of the present invention, the XRPD pattern analysis data of the crystal form A is shown in Table 1:

**Table 1. The XRPD pattern analysis data of the crystal form A**

| No. | **2θ (°)** | **Spacing (Å)** | **Relative intensity (%)** | **No.** | **2θ (°)** | **Spacing (Å)** | **Relative intensity (%)** |
|---|---|---|---|---|---|---|---|
| **1** | 3.33 | 26.55 | 24.00 | **13** | 17.04 | 5.20 | 41.36 |
| **2** | 4.84 | 18.27 | 83.58 | **14** | 18.04 | 4.92 | 31.46 |
| **3** | 5.09 | 17.37 | 100.00 | **15** | 19.16 | 4.63 | 50.31 |
| **4** | 7.13 | 12.39 | 33.00 | **16** | 19.95 | 4.45 | 24.06 |
| **5** | 9.35 | 9.45 | 52.44 | **17** | 20.26 | 4.38 | 28.68 |
| **6** | 10.17 | 8.70 | 23.08 | **18** | 20.70 | 4.29 | 16.05 |
| **7** | 11.98 | 7.39 | 32.16 | **19** | 21.71 | 4.09 | 13.38 |
| **8** | 13.24 | 6.69 | 68.09 | **20** | 22.52 | 3.95 | 14.95 |
| **9** | 14.35 | 6.17 | 46.81 | **21** | 24.43 | 3.64 | 8.35 |
| **10** | 14.62 | 6.06 | 35.96 | **22** | 26.11 | 3.41 | 8.34 |
| **11** | 15.38 | 5.76 | 34.78 | **23** | 28.41 | 3.14 | 5.80 |
| **12** | 15.86 | 5.59 | 60.60 | | | | |

In some embodiments of the present invention, a differential scanning calorimetry curve of the above crystal form A has an endothermic peak starting at 134.7°C ± 3° C.

In some embodiments of the present invention, the DSC curve of the above crystal form A is shown in Figure 2.

In some embodiments of the present invention, a thermal gravimetric analysis curve of the above crystal form A has a weight loss of 0.97% at 110°C ± 3° C.

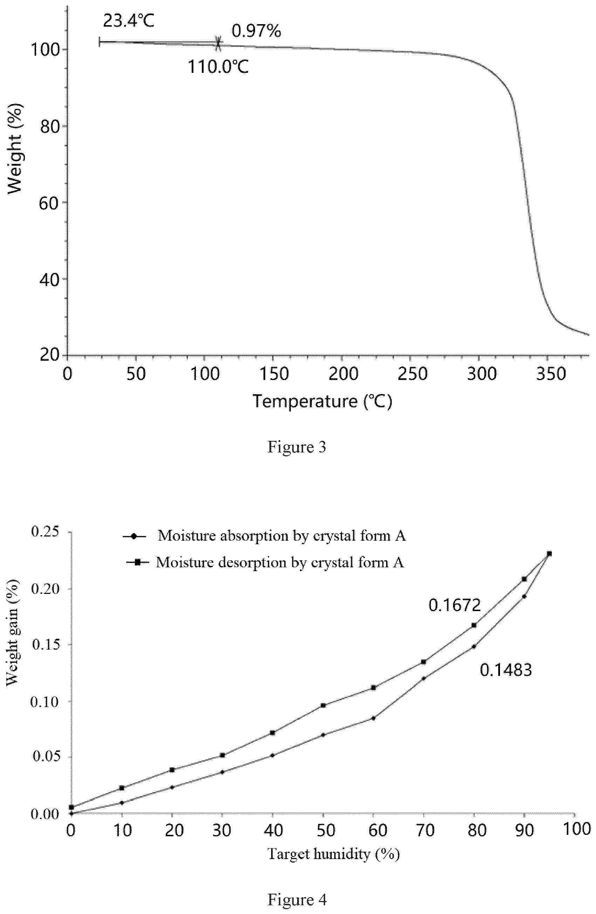

In some embodiments of the present invention, the TGA curve of the crystal form A is as shown in Figure 3.

The invention also provides a preparation method of the crystal form A of the compound represented by formula (I), comprising an anti-solvent addition method, a slow cooling method, a room temperature suspension stirring method, a gas-liquid permeation method, a slow volatilization method or a temperature cycling method.

In some embodiments of the present invention, the anti-solvent addition method comprises:
1) adding the compound represented by formula (I) into a solvent to form a saturated solution; and
2) adding an anti-solvent to the solution,
wherein the solvent is isopropanol, dichloromethane, tetrahydrofuran, acetone or ethyl acetate; and the anti-solvent is H₂O or n-pentane.

In some embodiments of the present invention, the room-temperature suspension stirring method described above comprises:
1) preparing a saturated solution at 50° C with the compound of formula (I) in a solvent; and
2) cooling the solution from 50°C to 5°C at a rate of 0.1°C per minute; and
wherein the solvent is a mixture of toluene and n-pentane at a volume ratio of 2: 1, ethyl acetate and n-pentane at a volume ratio of 4: 1, water and 1,4-dioxane at a volume ratio of 9: 1, methyl isobutyl ketone and n-pentane at a volume ratio of 3: 1, trichloromethane and 1-n-pentanol at a volume ratio of 1: 1 or N, N-dimethylformamide and H₂O at a volume ratio of 1: 1.

In some embodiments of the present invention, the slow cooling method comprises:
1) adding a compound of formula (I) to a solvent to form a suspension; and
2) carrying out crystal transformation by magnetically stirring the suspension; and
wherein the solvent is 2-butanol, H₂O, a mixture of isopropanol and H₂O at a volume ratio of 98: 2 to 85: 15, a mixture of n-pentane and acetone at a volume ratio of 9: 1, a mixture of n-pentane and ethanol at a volume ratio of 19: 1, a mixture of anisole and n-pentane at a volume ratio of 4: 1, a mixture of dimethyl sulfoxide and H₂O at a volume ratio of 3: 1, a mixture of methyl isobutyl ketone and n-butanol H at a volume ratio of 2: 1, a mixture of dichloromethane and n-pentane at a volume ratio of 1: 1, a mixture of 2-butanone and n-pentane at a volume ratio of 3: 1, a mixture of toluene and n-heptane at a volume ratio of 3: 1, a mixture of xylene and n-butanol at a volume ratio of 1: 1, a mixture of ethyl acetate and n-heptane at a volume ratio of 4: 1 or a mixture of methanol and H₂O at a volume ratio of 4: 1.

In some embodiments of the present invention, the gas-liquid permeation method comprises:
1) preparing a clear solution by adding the compound of formula (I) to anisole at room temperature; and
2) placing the clear solution in an atmosphere of n-pentane to allow well contacting of the n-pentane with the solution to induce crystallization.

In some embodiments of the present invention, the slow volatilization method comprises:
1) dissolving the compound of formula (I) in a solvent to form a clear solution; and
2) sealing the clear solution with a sealing film, and making holes in the sealing film to allow slow volatilization and crystallization; and
wherein the solvent is ethanol or a mixture of ethanol and H₂O at a volume ratio of 4: 1.

In some embodiments of the present invention, the temperature cycling method comprises:
1) dissolving the compound of formula (I) in a solvent to form a turbid solution of 50°C; and
2) carrying out crystal transformation by circulating the turbid solution at a speed of 0.1° C/minute according to a procedure of 50°C to 5° C; and
wherein the solvent is n-pentane, H₂O, a mixture of acetone and H₂O at a volume ratio of 4: 1, a mixture of 2-butanone and n-heptane at a volume ratio of 3: 1, a mixture of isopropyl acetate and n-butanol at a volume ratio of 1: 1 or a mixture of xylene and n-heptane at a volume ratio of 4: 1.

The present invention also provides a preparation method of the crystal form A of the compound represented by formula (I), comprising:
1) adding acetone to the compound of formula (I) to dissolve; and
2) adding n-heptane, stirring at room temperature for 3-4 hours, and filtering.

The invention also provides use of the crystal form A in the manufacture of a medicament for treating a SGLT1/SGLT2 related disease.

### Technical effects

The crystal form A of the compound represented by formula (I) of the present invention is stable and less affected by light, heat or humidity, has a good in vivo drug administration effect, and has a broad market prospect.

### Definition and description

Unless otherwise indicated, the following terms and phrases as used herein are intended to have the following meanings. A particular phrase or term should not be considered undefined or unclear without particular definitions but should be understood in accordance with the ordinary meaning. When a commodity name appears herein, it is intended to refer to its corresponding commodity or its active ingredient.

The intermediate compounds of the present invention may be prepared by a variety of synthetic methods well known to those skilled in the art, including the specific embodiments listed below, the embodiments formed in conjunction with other chemical synthesis methods, and equivalent alternatives well known to those skilled in the art. Preferred embodiments include but are not limited to the embodiments of the present disclosure.

The chemical reactions in the specific embodiments of the present invention are accomplished in a suitable solvent which is suitable for the chemical changes of the present invention and the required reagents and materials. In order to obtain the compounds of the present invention, it is sometimes necessary for a person skilled in the art to modify or select a synthesis step or a reaction process based on an existing embodiment.

Structures of the compounds of the present invention may be confirmed by conventional methods well known to those skilled in the art, and if the present invention relates to the absolute configuration of the compounds, the absolute configuration may be confirmed by conventional technical means in the art. For example, single crystal X-ray diffraction (SXRD) may be used, wherein a cultured single crystal is analyzed by a Bruker D8 Venture diffractometer to collect diffraction intensity data, and the light source is CuKα radiation, and the scanning mode is ϕ/ω scanning, and relevant data are collected and the crystal structure is further analyzed by using a direct method (Shelxs97), so that the absolute configuration can be confirmed.

Hereinafter, the present disclosure is described in detail below by referring to the examples, which are not intended to adversely limit the present disclosure.

All solvents used in the present invention are commercially available and can be used without further purification.

Solvents used in the present invention can be commercially available. The present invention employs the following abbreviations: EtOH represents ethanol; MeOH represents methanol; TFA represents trifluoroacetic acid; TsOH represents p-toluenesulfonic acid; mp represents a melting point; EtSO₃H represents ethanesulfonic acid; MeSO₃H represents methanesulfonic acid; THF represents tetrahydrofuran; and EtOAc represents ethyl acetate.

### X-ray powder diffractometer (XRPD) method of the present disclosure

Instrument Model: X'Pert³ type X-ray Diffractometer, PANalytical Company

Test method: about 10 mg of sample is used for XRPD detection.

Detailed XRPD parameters are as follows:
Ray source: Cu, Kα (Kα1=1.540598 Ǻ, Kα2=1.544426 Å, strength ratio Kα2/ Kα1= 0.5)
Voltage of the X-Ray tube: 45 kV, current of the X-Ray tube: 40 mA
Divergent slit: Fixed 1/8 Deg
First soller slit: 0.04 rad, Second soller slit: 0.04 rad
Receiving slit: No, Anti-scatter slit: 7.5 mm
Measurement time: 5 min
Scanning angle range: 3-40 deg
Step width angle: 0.0263 deg
Step size: 46.665 seconds
Sample disc rotation speed: 15 rpm

### Differential Scanning Calorimeter (DSC) method of the present invention

Instrument model: TA Q200/Q2000/2500 differential scanning calorimeter

Test method: A sample (about 1-5 mg) is placed in a DSC aluminum disk, and heated from 25°C (room temperature) to the sample decomposition at a heating rate of 10° C/min under a 50mL/min nitrogen condition.

### Thermal Gravimetric Analyzer (TGA) method of the present invention

Instrument Model: TA Q5000/5500 thermal gravimetric analyzer

Test method: A sample (about 1-5 mg) is placed in a TGA aluminum disk, and heated from room temperature to 350°C at a heating rate of 10° C/min under a nitrogen condition of 10mL/min.

### Dynamic Vapor Sorption (DVS) method of the present invention

Instrument Model: SEM Advantage-1 dynamic vapor adsorber

Test conditions: Sample (10-30 mg) is placed in the DVS sample tray for testing.

Detailed DVS parameters are as follows:
Temperature: 25°C
Balance: dm/dt = 0.002%/min (shortest: 10 min, longest: 180 min)
RH (%) test step: 10 (0-90%), 5 (90-95%)
RH (%) test step range: 0 - 95 - 0
Hygroscopicity evaluation and classification are as follows:

| **Classification of hygroscopicity** | ΔW % |
|---|---|
| Deliquescence | Absorbing enough water to form a liquid |
| Very hygroscopic | ΔW% ≥ 15% |
| hygroscopic | 15% > ΔW% ≥ 2% |
| Slightly hygroscopic | 2% > ΔW% ≥ 0.2% |
| No or substantially no hygroscopicity | ΔW% < 0.2% |

| | |
|---|---|
| Note: ΔW % indicates weight gain caused by hygroscopicity at 25°C and 80% RH. | |

### BRIEF DESCRIPTION OF THE DRAWINGS

Figure 1 is an XRPD pattern using Cu-Kα radiation of the crystal form A of a compound represented by formula (I);
Figure 2 is a DSC curve of the crystal form A of a compound represented by formula (I);
Figure 3 is a TGA curve of the crystal form A of a compound represented by formula (I);
Figure 4 is a DVS curve of the crystal form A of a compound represented by formula (I).

### DETAILED DESCRIPTION

In order to better understand the content of the present disclosure, the present disclosure is further described in detail below by referring to the examples, which are not intended to adversely limit the present disclosure.

### Example 1: Preparation of a compound represented by formula (I)

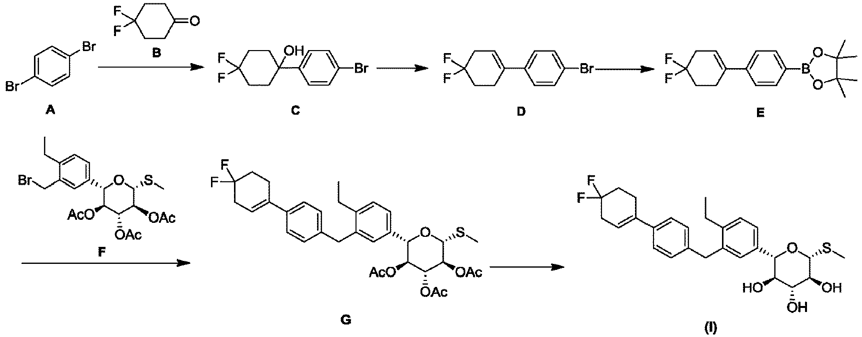

### Step 1: Synthesis of Compound C

Tetrahydrofuran (3.2L) and compound A (400.12 g, 1.70 mol, 1 eq) are sequentially added to a pre-dried three-necked flask (10L). After replacing with nitrogen, an internal temperature thereof is cooled to less than -60° C. Then n-butyllithium (2.5 M, 712 mL, 1.05 eq) is slowly added dropwise thereto, stirred for 0.5 hours, then Compound B (250.00 g, 1.86 mol, 1.1 eq) in tetrahydrofuran (250 mL) is added dropwise, and the resulted solution is slowly warmed to 0°C and stirred for 0.5 hours. After the reaction is completed, the reaction solution is slowly poured into water (3L) at 0-5°C for quenching, then stirred for 10 minutes, and allowed to separate into layers by standing, wherein an organic phase is reserved, and an aqueous phase is extracted with ethyl acetate (2 L X 2). Organic phases are merged, washed with saturated sodium chloride solution (1 L), and concentrated under reduced pressure to obtain a crude compound C which will be directly used for the next reaction.

### Step 2: Synthesis of Compound D

Compound C (493.04 g, 1.70 mol, 1 eq), acetic acid (2L) and concentrated sulfuric acid (16.56 g, 0.17 mol, 0.1 eq) are sequentially added to a pre-dried three-necked flask (5L), heated to 100°C and stirred for 1 hour. After the reaction is completed, the solvent is evaporated under reduced pressure. The residue is dissolved in ethyl acetate (3L), neutralized with 0.5M NaOH, and then the organic phase is washed successively with saturated NaHCO₃ solution (1 L) and saturated NaCl solution (0.5 L), followed by reduced pressure concentration to obtain a crude product. Methanol (5740 mL) is added to the crude product, heated to 40°C to allow well dispersion of the system, then cooled to room temperature, stirred for 4 hours, and filtered. The filtrate is concentrated under reduced pressure to obtain a crude product, into which methanol (1150 mL) is added and heating is performed to dissolve. The resulted solution is cooled to room temperature, stirred for 2 hours, and filtered by suction to obtain a compound D. ¹H NMR (400 MHz, CHCl₃-d) δ ppm 7.43 - 7.49 (m, 2 H), 7.21 - 7.27 (m, 2 H), 5.88 - 5.95 (m, 1 H), 2.64 - 2.77 (m, 4 H), 2.12 - 2.26 (m, 2 H).

### Step 3: Synthesis of Compound E

To a pre-dried 50 L reaction kettle, 1,4-dioxane (22L) is added, then compound D (2200.00 g, 4.02 moL, 1 eq), bis(pinacolato)diboron (2147.20 g, 4.22 moL, 1.05 eq), potassium acetate (1185.80 g, 5.73 moL, 1.5 eq) and [1,1'-bis(diphenylphosphino)ferrocene]dichloropalladium (58.80 g, 0.03 moL, 0.01 eq) are successively added thereto, heated under nitrogen protection to 100°C and reacted for 5 hours. The resulted reaction solution is concentrated under reduced pressure and then dissolved in ethyl acetate (22L), water (22L) is added thereto to perform washing, and the aqueous phase is extracted with ethyl acetate (11L). Organic phases are merged and concentrated under reduced pressure to obtain a crude product. N-heptane (3.5L) is added to the crude product, stirred at room temperature for 1 hour, filtered, and the filter cake is collected and dried under vacuum for 16 hours to give a compound E. ¹H NMR (400 MHz, CHCl₃-d) δ ppm 7.78 (d, J=8.3 Hz, 2 H), 7.38 (d, J=8.1 Hz, 2 H), 5.94 - 6.00 (m, 1 H), 2.67 - 2.79 (m, 4 H), 2.19 (tt, J=13.7, 6.6 Hz, 2 H), 1.36 (s, 12 H).

### Step 4: Synthesis of Compound G

To a pre-dried 50 L reaction kettle, 1,4-dioxane (12L) and water (3L) are added, then compound E (1493.00 g, 2.96 mol, 1.0 eq), compound F (1139.45 g, 3.55 mol, 1.2 eq), potassium carbonate (614.78 g, 4.44 mol, 1.5 eq) and tetrakis(triphenylphosphine)palladium (171.43 g, 0.14 mol, 0.05 eq) are successively added thereto, and stirred under nitrogen protection at 50°C for 16 hours. After the reaction is cooled, ethyl acetate (7.5L) and water (7.5L) are added thereto and stirred, followed by standing to separate into layers. The separated aqueous phase is extracted with ethyl acetate (7.5L). Organic phases are merged and concentrated under reduced pressure to obtain a crude product.Ethanol (15L) is added to the crude product, heated to 80°C and stirred until the solution is clear. Then the solution is cooled to 30°C and stirred for 2 hours at this temperature, filtered, and the filter cake is collected. The filter cake is subjected to palladium removal to give a compound G. ¹H NMR (400 MHz, CHCl₃-d) δ ppm 7.29 (s, 1 H), 7.26 (s, 1 H), 7.17 - 7.22 (m, 2 H), 7.01 - 7.06 (m, 3 H), 5.88 (br s, 1 H), 5.29 - 5.36 (m, 1 H), 5.18 - 5.24 (m, 1 H), 5.12 (t, J=9.7 Hz, 1 H), 4.52 (d, J=9.8 Hz, 1 H), 4.39 (d, J=9.8 Hz, 1 H), 3.93 - 4.05 (m, 2 H), 2.65 - 2.76 (m, 4 H), 2.58 (q, J=7.5 Hz, 2 H), 2.13 - 2.24 (m, 5 H), 2.10 (s, 3 H), 2.01 (s, 3 H), 1.73 (s, 3 H), 1.55 (s, 3 H), 1.13 (t, J=7.5 Hz, 3 H).

### Step 5: Synthesis of compound of formula (I)

Anhydrous methanol (8.5L) is added to a pre-dried 50 L reaction kettle, then compound G (855.83 g, 1.42 moL, 1 eq) and 25% sodium methoxide in methanol solution (171 mL) are successively added thereto. The resulted reaction solution is stirred at room temperature for 16 hours. After the reaction is completed, standing and filtering are performed. Water (8.5L) is added into the filtrate, heated to 40° C and stirred for 2 hours, and then stirred at room temperature for 16 hours. Then filtration is carried out and a solid crude product is collected. Anhydrous dichloromethane (11L) is added to the crude product, followed by standing to allow separating into layers, and the organic phase is collected and concentrated to obtain the compound of formula (I). ¹H NMR (400 MHz, CH₃OH-d₄) δ ppm 7.32 (d, J=8.3 Hz, 2 H), 7.23 - 7.27 (m, 1 H), 7.17 - 7.22 (m, 2 H), 7.12 (d, J=8.3 Hz, 2 H), 5.92 (br s, 1 H), 4.40 (d, J=9.5 Hz, 1 H), 4.15 (d, J=9.0 Hz, 1 H), 4.03 (s, 2 H), 3.36 - 3.51 (m, 3 H), 2.58 - 2.74 (m, 6 H), 2.10 - 2.24 (m, 5 H), 1.11 (t, J=7.5 Hz, 3 H).

### Example 2: Preparation of Crystal Form A of Compound Represented by Formula (I)

**Table 2 Chinese and English names of solvents**

| **Reagents** | **English name** | **Reagents** | **English name** |
|---|---|---|---|
| Methanol | MeOH | N, N-dimethylformamide | DMF |
| Ethanol | EtOH | Anisole | Anisole |
| Isopropanol | IPA | n-heptane | n-Heptane |
| Acetone | Acetone | Toluene | Toluene |
| Methyl isobutyl ketone | MIBK | Dichloromethane | DCM |
| Ethyl acetate | EtOAc | Dimethyl sulfoxide | DMSO |
| Isopropyl acetate | IPAc | Water | H₂O |
| Tetrahydrofuran | THF | 2-Butanone | MEK |
| 1, 4-Dioxane | 1,4-dioxane | 2-Butanol | 2-BuOH |
| n-hexane | n-Hexane | Chloroform | CHCl₃ |
| n-butanol | 1-BuOH | Xylene | Xylene |
| 1-pentanol | 1-pentanol | n-pentane | n-Pentane |

### Method 1: Anti-solvent addition method

Nearly saturated solutions in different solvents are prepared respectively with approximately 15 mg of the compound of formula (I), and a corresponding anti-solvent is added into each solution. The obtained solids are separated and dried to perform XRPD tests. The experimental results are as follows:

| Experiment No. | Solvents | Anti-solvents | Crystal form of solid |
|---|---|---|---|
| 1 | IPA | H₂O | Crystal form A |
| 2 | DCM | N-Heptane | Crystal form A |
| 3 | THF | N-Heptane | Crystal form A |
| 4 | Acetone | N-Pentane | Crystal form A |
| 5 | EtOAc | N-Pentane | Crystal form A |

### Method 2: Slow cooling method

Nearly saturated solutions of 50°C in different solvents are prepared respectively with approximately 15 mg of the compound of formula (I), cooled from 50°C to 5°C at a rate of 0.1°C per minute, and resulted solids are collected to preform XRPD tests. The experimental results are as follows:

| Experiment No. | Solvents (v/v) | Crystal form of solids |
|---|---|---|
| 1 | Toluene/n-Heptane, 2:1 | Crystal form A |
| 2 | EtOAc/n-Pentane, 4:1 | Crystal form A |
| 3 | H₂O/1,4-Dioxane, 9:1 | Crystal form A^{#} |
| 4 | MIBK/n-Pentane, 3:1 | Crystal form A |
| 5 | CHCl₃/1-Pentanol, 1:1 | Crystal form A |

| | | |
|---|---|---|
| Note: for "#" samples, a large amount of solids of crystal form A are generated when equilibrated at 50° C. | | |

### Method 3: Room temperature suspension stirring method

Suspensions are prepared respectively with approximately 15 mg of the compound of formula (I) in different solvents. The suspensions are magnetically stirred to allow crystal transformation, then solids are collected by centrifugation and XRPD tests are performed. The experimental results are as follows:

| Experiment No. | Solvents (v/v) | Crystal form of Solids |
|---|---|---|
| 1 | 2-BuOH | Crystal form A |
| 2 | n-Pentane/Acetone, 9:1 | Crystal form A |
| 3 | n-Heptane/EtOH, 19:1 | Crystal form A |
| 4 | Anisole/n-Pentane, 4:1 | Crystal form A |
| 6 | MIBK/1-BuOH, 2:1 | Crystal form A |
| 7 | DCM/n-Pentane, 1:1 | Crystal form A |
| 8 | MEK/n-Pentane, 3:1 | Crystal form A |
| 9 | IPA/H₂O (98:2) | Crystal form A |
| 10 | IPA/H₂O (95:5) | Crystal form A |
| 11 | IPA/H₂O (92:8) | Crystal form A |
| 12 | IPA/H₂O (85:15) | Crystal form A |
| 13 | H₂O | Crystal form A |
| 14 | Toluene/n-Heptane, 3:1 | Crystal form A |
| 15 | Xylene/1-BuOH, 1:1 | Crystal form A |
| 16 | EtOAc/n-Heptane, 4:1 | Crystal form A |
| 17 | MeOH/H₂O, 4:1 | Crystal form A |

### Method 4: Gas-liquid permeation method

A clear solution is prepared respectively with approximately 15 mg of the compound of formula (I) in a solvent at room temperature. The clear solution is placed in an atmosphere of an anti-solvent, so that the anti-solvent vapor and the solution are full contacted with to induce crystallization. The obtained solid is collected and XRPD test is carried out, and the experimental results are as follows:

| Experiment No. | Solvent | Anti-solvent | Crystal form of solid |
|---|---|---|---|
| 1 | Anisole | n-Pentane | Crystal form A |

### Method 5: Slow volatilization method

Clear solutions are prepared respectively with approximately 15 mg of the compound of formula (I) in different solvents. Each of the clear solutions is sealed with a sealing film, and holes are made in the sealing film to allow slow volatilization and crystallization. The resulted solids are collected and XRPD tests are performed. The experimental results are as follows:

| Experiment No. | Solvents | Crystal form of solids |
|---|---|---|
| 1 | EtOH | Crystal form A |
| 2 | EtOH/H₂O, 4: 1 | Crystal form A |

### Method 6: Temperature cycling method

Suspensions are prepared respectively with approximately 15 mg of the compound of formula (I) in different solvents. The suspensions are circulated at a speed of 0.1° C/minute according to a procedure of 50°C to 5° C to allow crystal transformation. The resulting solids are collected and XRPD tests are performed. The experimental results are as follows:

| Experiment No. | Solvents | Crystal form of solids |
|---|---|---|
| 1 | n-Heptane | Crystal form A |
| 2 | H₂O | Crystal form A |
| 3 | Acetone/H₂O, 4:1 | Crystal form A |
| 4 | MEK/n-Heptane, 3:1 | Crystal form A |
| 5 | IPAc/1-BuOH, 1:1 | Crystal form A |
| 6 | Xylene/n-Hexane, 4:1 | Crystal form A |

### Method 7:

371 g of the compound of formula (I) is dissolved in acetone (530mL), and n-heptane (4770mL) is added thereto. The resulted solution is stirred at room temperature for 3 hours, then filtered and a filter cake is collected, obtaining the crystal form A of formula (I). ¹H NMR (400 MHz, CH₃OH-d₄) δ ppm 7.32 (d, J=8.3 Hz, 2 H), 7.23 - 7.27 (m, 1 H), 7.17 - 7.22 (m, 2 H), 7.12 (d, J=8.3 Hz, 2 H), 5.92 (br s, 1 H), 4.40 (d, J=9.5 Hz, 1 H), 4.15 (d, J=9.0 Hz, 1 H), 4.03 (s, 2 H), 3.36 - 3.51 (m, 3 H), 2.58 - 2.74 (m, 6 H), 2.10 - 2.24 (m, 5 H), 1.11 (t, J=7.5 Hz, 3 H).

### Example 3: Hygroscopicity study of crystal form A of compound of formula (I)

Experimental Material:
SMS DVS Advantage Dynamic Vapor Sorption Intrinsic

Experimental Method:
10-30 mg of the crystal form A of compound of formula (I) is placed in a DVS sample tray for testing.

Experimental results:
The DVS pattern of the crystal form A of compound of formula (I) is shown in Figure 4, ΔW = 0.1483%.

Experimental Conclusion:
The crystal form A of compound of formula (I) has a weight gain of 0.1483% caused by hygroscopicity at 25°C and 80% RH, indicating it has almost no hygroscopicity.

### Example 4: Stability Test of crystal form A of compound of formula (I)

12 parts of crystal form A of compound of formula (I), approximately 5 mg per part, are weighed out. Each part is placed at the bottom of a HPLC vial and spread into a thin layer. Samples placed in a constant temperature and constant humidity box at 60° C/75%RH and samples placed in a 92.5% RH dryer are sealed with a sealing film on the bottle mouth, and some small holes are made in the sealing film to ensure that the sample can be brought into full contact with the ambient air. Samples placed at 60° C, or under lights or shading conditions are tightly capped (wherein the samples under shading conditions are packaged with a tin foil paper). The test results are shown in Table 3 below:

**Table 3 Solid stability test results of Compound A crystal form of Formula (I)**

| **Conditions** | **Test time** | **Purity (area%)** | **Crystal form** |
|---|---|---|---|
| 0 day | - | 96.48 | Crystal form A |
| 60 °C | 5 days | 96.38 | Crystal form A |
| | 10 days | 96.41 | Crystal form A |
| 92.5%RH | 5 days | 96.41 | Crystal form A |
| | 10 days | 96.47 | Crystal form A |

| Visible lights^{#} | illuminance reaches 1.2E+06 Lux·hrs | 96.29 | Crystal form A |
|---|---|---|---|
| Shading lights | Test at the same time with the visible lights group | 96.44 | Crystal form A |
| Visible lights + Ultraviolet lights ^{#} | illuminance reaches 200 W·hrs/m² | 95.75 | Crystal form A |
| Shading lights | Test at the same time with the visible lights + ultraviolet lights group | 96.43 | Crystal form A |
| 60 °C/75%RH | 1 months | 96.31 | Crystal form A |
| | 2 months | 96.39 | Crystal form A |
| | 3 months | 96.37 | Crystal form A |

| | | | |
|---|---|---|---|
| Note: "#" indicates compliance with ICH requirements. | | | |

Conclusion: The crystal forms A of compound of formula (I) has good stability.

### Example 5: Pharmacodynamic study of crystal form A of compound of Formula (I) in rats OGTT model

Experimental Material:
SD rats, male, aged 7-8 weeks, purchased from Zhejiang Weitonglihua Experimental Animal Technology Co. Ltd. are maintained in a special pathogen-free environment, with 4 rats in a single ventilation cage. Beddings and water in all cages are sterilized prior to use. All animals can freely obtain a standard certified commercial laboratory diet.

Experimental Method:
After the animals are adapted to the environment for one week, they are fasted for 16 hours in a metabolic cage, and randomly divided into different groups according to fasting blood glucose and body weight. The animals are administered with a drug or solvent (2 mL/kg), followed by immediately administration of 50% glucose solution (2 g/kg, 4 mL/kg). 2 hours after the administration of glucose solution, the feed is recovered, and blood samples are collected at 0 min, 15 min, 30 min, 45 min, 60 min, and 120 min for blood glucose testing. All values are expressed as average values.

Statistical analysis is carried out using Graphpad Prism 6 double factor variance analysis and evaluated with Tukey's multiple comparison tests. P values less than 0.05 are considered to have statistical significance.

### Experimental results: See Table 4

**Table 4 Experimental results for rat sugar tolerance**

| Compounds | Vehicle control group | Positive compound Sotagliflozin (3 mg/Kg) | Crystal form A of compound of formula (I) | | |
|---|---|---|---|---|---|
| | | | (1mg/kg) | (3 mg/kg) | (10 mg/kg) |
| OGTT Blood glucose levels AUC₀₋₂ₕᵣ (mol/L×min) | 1167.8±30.24 | 1046.7±33.17* | 1065.6±34 .95 | 977.1±36. 91*** | 881.4±14.75 **** |

| | | | | | |
|---|---|---|---|---|---|
| Notes: 1. Data is expressed as mean ± standard error. 2. Statistical analysis is performed using two-Way ANOVA, comparing with the control group, * denotes p < 0.05, * * denotes p < 0.001, * * * denotes p < 0.001, * * * * denotes p < 0.0001. | | | | | |

Experimental Conclusion:
The crystal form A of compound of formula (I) can effectively and significantly reduce the blood glucose level, AUC₀₋₂ₕᵣ, in 2 hours of animals.

## Claims

1. Crystal form A of a compound represented by formula (I), wherein an X-ray powder diffraction pattern of the crystal form A has characteristic diffraction peaks at the following 2θ angles: 5.09 ± 0.20°, 9.35 ± 0.20°, and 13.24 ± 0.20°

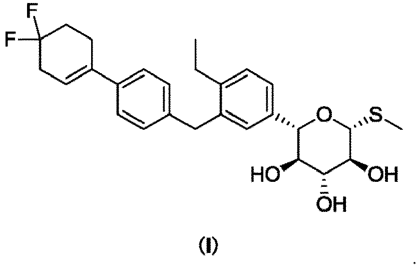

2. The crystal form A according to claim 1, wherein the X-ray powder diffraction pattern has characteristic diffraction peaks at the following 2θ angles: 5.09 ± 0.20°, 9.35 ± 0.20°, 13.24 ± 0.20°, 14.35 ± 0.20°, 15.86 ± 0.20°, 17.04 ± 0.20°, 18.04 ± 0.20°, and 19.16 ± 0.20°.

3. The crystal form A according to claim 2, wherein the X-ray powder diffraction pattern has characteristic diffraction peaks at the following 2θ angles: 5.09 ± 0.20°, 7.13 ± 0.20°, 9.35 ± 0.20°, 11.98 ± 0.20°, 13.24 ± 0.20°, 14.35 ± 0.20°, 15.86 ± 0.20°, 17.04 ± 0.20°, 18.04 ± 0.20°, and 19.16 ± 0.20°.

4. The crystal form A according to claim 3, wherein the X-ray powder diffraction pattern has characteristic diffraction peaks at the following 2θ angles: 3.33°, 4.84°, 5.09°, 7.13°, 9.35°, 10.17°, 11.98°, 13.24°, 14.35°, 14.62°, 15.38°, 15.86°, 17.04°, 18.04°, 19.16°, 19.95°, 20.26°, 20.70°, 21.71°, 22.52°, 24.43°, 26.11° and 28.41°.

5. The crystal form A according to claim 4, wherein the XRPD pattern of the crystal form A is as shown in Figure 1.

6. The crystal form A according to any one of claims 1 to 5, wherein a differential scanning calorimetry curve of the crystal form A has an endothermic peak starting at 134.7 ± 3° C.

7. The crystal form A according to claim 6, wherein the DSC curve is as shown in Figure 2.

8. The crystal form A according to any one of claims 1 to 5, wherein a thermal gravimetric analysis curve of the crystal form A has a weight loss of 0.97% at 110 ± 3° C.

9. The crystal form A according to claim 8, wherein the TGA curve is as shown in Figure 3.

10. A preparation method for a crystal form A of the compound represented by formula (I), comprising an anti-solvent addition method, a slow cooling method, a room temperature suspension stirring method, a gas-liquid permeation method, a slow volatilization method, or a temperature cycling method.

11. The preparation method according to claim 10, wherein the anti-solvent addition method comprises:
1) adding the compound represented by formula (I) into a solvent to form a saturated solution; and
2) adding an anti-solvent to the solution,
wherein the solvent is isopropanol, dichloromethane, tetrahydrofuran, acetone or ethyl acetate; and the anti-solvent is H₂O or n-pentane.

12. The preparation method according to claim 10, wherein the room temperature suspension stirring method comprises:
1) preparing a saturated solution at 50° C with the compound of formula (I) in a solvent; and
2) cooling the solution from 50°C to 5°C at a rate of 0.1°C per minute; and
wherein the solvent is a mixture of toluene and n-pentane at a volume ratio of 2: 1, ethyl acetate and n-pentane at a volume ratio of 4: 1, water and 1,4-dioxane at a volume ratio of 9: 1, methyl isobutyl ketone and n-pentane at a volume ratio of 3: 1, trichloromethane and 1-n-pentanol at a volume ratio of 1: 1 or N, N-dimethylformamide and H₂O at a volume ratio of 1: 1.

13. The preparation method according to claim 10, wherein the slow cooling method comprises:
1) adding a compound of formula (I) to a solvent to form a suspension; and
2) carrying out crystal transformation by magnetically stirring the suspension; and
wherein the solvent is 2-butanol, H₂O, a mixture of isopropanol and H₂O at a volume ratio of 98: 2 to 85: 15, a mixture of n-pentane and acetone at a volume ratio of 9: 1, a mixture of n-pentane: ethanol at a volume ratio of 19: 1, a mixture of anisole and n-pentane at a volume ratio of 4: 1, a mixture of dimethyl sulfoxide and H₂O at a volume ratio of 3: 1, a mixture of methyl isobutyl ketone and n-butanol H at a volume ratio of 2: 1, a mixture of dichloromethane and n-pentane at a volume ratio of 1: 1, a mixture of 2-butanone and n-pentane at a volume ratio of 3: 1, a mixture of toluene and n-heptane at a volume ratio of 3: 1, a mixture of xylene and n-butanol at a volume ratio of 1: 1, a mixture of ethyl acetate and n-heptane at a volume ratio of 4: 1 or a mixture of methanol and H₂O at a volume ratio of 4: 1.

14. The preparation method according to claim 10, wherein the gas-liquid permeation method comprises:
1) preparing a clear solution by adding the compound of formula (I) to anisole at room temperature; and
2) placing the clear solution in an atmosphere of n-pentane to allow well contacting of the n-pentane with the solution to induce crystallization.

15. The preparation method according to claim 10, wherein the slow volatilization method comprises:
1) dissolving the compound of formula (I) in a solvent to form a clear solution; and
2) sealing the clear solution with a sealing film, and making holes in the sealing film to allow slow volatilization and crystallization; and
wherein the solvent is ethanol or a mixture of ethanol and H₂O at a volume ratio of 4: 1.

16. The preparation method according to claim 10, wherein the temperature cycling method comprises: 1) dissolving the compound of formula (I) in a solvent to form a turbid solution of 50°C; and
2) carrying out crystal transformation by circulating the turbid solution at a speed of 0.1° C/minute according to a procedure of 50°C to 5° C; and
wherein the solvent is n-pentane, H₂O, a mixture of acetone and H₂O at a volume ratio of 4: 1, a mixture of 2-butanone and n-heptane at a volume ratio of 3: 1, a mixture of isopropyl acetate and n-butanol at a volume ratio of 1: 1 or a mixture of xylene and n-heptane at a volume ratio of 4: 1.

17. A preparation method for crystal form A of a compound represented by formula (I), comprising:
1) adding acetone to the compound of formula (I) to dissolve; and
2) adding n-heptane, stirring at room temperature for 3-4 hours, and filtering.

18. Use of the crystal form A according to any one of claims 1-9 or the crystal form A obtained by the preparation method according to any one of claims 10-17 in the manufacture of a medicament for treating an SGLT1/SGLT2 related disease.
